# EUROPEAN PATENT SPECIFICATION

(11) **EP 1 637 590 B1**
(45) Date of publication and mention of the grant of the patent: **19.10.2011**
(21) Application number: 04734379.3
(22) Date of filing: 21.05.2004
(51) Int. Cl.: C12N 5/074

(54) **DIFFERENTIATED CELLS ORIGINATING IN PRECURSOR FAT CELLS AND METHOD OF ACQUIRING THE SAME**
DIFFERENZIERTE ZELLEN MIT URSPRUNG IN VORLÄUFERFETTZELLEN SOWIE VERFAHREN ZUR GEWINNUNG DAVON
CELLULES DIFFERENTIEES ISSUES DE PRECURSEURS D'ADIPOCYTES ET LEUR PROCEDE D'OBTENTION

(30) Priority: 13.06.2003 JP 2003170011
(43) Date of publication of application: 22.03.2006
(73) Proprietor: School Juridical Person Nihon University, Tokyo 102-8275 (JP)
(72) Inventor: KANO, Koichiro, c/o Nihon University, Tokyo 1028275 (JP)
(74) Representative: Wakerley, Helen Rachael
(86) International application number: PCT/JP2004/007322
(87) International publication number: WO 2004/111211

(56) References cited:
- WO-A-99/28444
- JP-A- 2000 083 656
- JP-A- 2002 537 849
- ZUK P A ET AL: "Human adipose tissue is a source of multipotent stem cells" MOLECULAR BIOLOGY OF THE CELL, BETHESDA, MD, US, vol. 13, no. 12, 20 December 2002 (2002-12-20), pages 4279-4295, XP002249630 ISSN: 1059-1524
- SAFFORD KRISTINE M ET AL: "Neurogenic differentiation of murine and human adipose-derived stromal cells" BIOCHEMICAL AND BIOPHYSICAL RESEARCH COMMUNICATIONS, vol. 294, no. 2, 7 June 2002 (2002-06-07), pages 371-379, XP002384763 ISSN: 0006-291X
- ZANGANI DANILO ET AL: "Adipocyte-epithelial interactions regulate the in vitro development of normal mammary epithelial cells" EXPERIMENTAL CELL RESEARCH, SAN DIEGO, CA, US, vol. 247, no. 2, 1999, pages 399-409, XP002306532 ISSN: 0014-4827
- ZUK P A ET AL: "Multilineage cells from human adipose tissue: Implications for cell-based therapies" TISSUE ENGINEERING, LARCHMONT, NY, US, vol. 7, no. 2, April 2001 (2001-04), pages 211-228, XP002198710 ISSN: 1076-3279
- VAN ROBIN L R ET AL: "CYTOLOGICAL AND ENZYMOLOGICAL CHARACTERIZATION OF ADULT HUMAN ADIPOCYTE PRECURSORS IN CULTURE" JOURNAL OF CLINICAL INVESTIGATION, vol. 58, no. 3, 1976, pages 699-704, XP002384764 ISSN: 0021-9738
- SUGIHARA H ET AL: "A SIMPLE CULTURE METHOD OF FAT CELLS FROM MATURE FAT TISSUE FRAGMENTS" JOURNAL OF LIPID RESEARCH, vol. 30, no. 12, 1989, pages 1987-1996, XP002384765 ISSN: 0022-2275
- SUGIHARA H ET AL: "PROLIFERATION OF UNILOCULAR FAT CELLS IN THE PRIMARY CULTURE" JOURNAL OF LIPID RESEARCH, vol. 28, no. 9, 1987, pages 1038-1045, XP002384766 ISSN: 0022-2275
- SUGIHARA H ET AL: "PRIMARY CULTURES OF UNILOCULAR FAT CELLS: CHARACTERISTICS OF GROWTHIN VITRO AND CHANGES IN DIFFERENTIATION PROPERTIES" DIFFERENTIATION, SPRINGER VERLAG, DE, vol. 31, 1986, pages 42-49, XP000990058 ISSN: 0301-4681
- SODA R ET AL: "ADIPOCYTE STEM CELL: A BRIEF REVIEW" INTERNATIONAL JOURNAL OF CELL CLONING, ALPHAMED PRESS, DAYTON, OH, US, vol. 1, no. 2, 1983, pages 79-84, XP009007124 ISSN: 0737-1454

## Description

### [Technical Field]

The present invention relates to a method of acquiring cells having other functions by inducing transdifferentiation of preadipocyte cell line. The cell line was obtained by dedifferentiated mature adipocytes derived from animal and human fat tissues. And the present invention relates to cells acquired by the method.

### [Background Art]

Examples of a method of exchanging the lost or failed part in a human body include: a method of compensating the part with an artifact such as artificial leg or tooth; and a method of transplanting a tissue such as skin or cornea using a part of somebody else's body. Transplant of organs such as kidney, heart, and lung became widely used from 19th century to 20th century. Among these substitutes, an artificial kidney (dialyzer) has been developed as a substitute including an artifact from about 1945 and is widely used today. Although artificial respirators, artificial hearts, or the like were also made for carrying out a part of heart or lung functions, most of them are apparatus to be used outside the body, so that there are many constraints on using them. Meanwhile, for the liver having complicated functions, it will be less likely to develop an artificial liver. Therefore, it is difficult to completely compensate vital functions by substituting organs with artifacts.

Organs such as the kidney, heart, and liver have various functions, and if those organs do not carry out the original functions, a human will die. Accordingly, in order to compensate for original vital functions completely, treatment has been performed by transplanting an organ derived from a living body to exchange a nonfunctional organ for a healthy organ derived from another human or animals. Transplant of an organ derived from a living body enabled compensation of vital functions in the heart and lung, which were difficult to be substituted for artifacts, as well as in the liver, which was nearly impossible to be developed using an artifact. Patients receiving organ transplant operations are increasing year by year. 700 or more kidney transplants per year, and 400 or more liver transplants per year are performed in Japan. Meanwhile, although the number of heart or lung transplant is still small, the survival rates of the patients are significantly enhanced. And the organ transplant has been established as an effective treatment method.

However, it is highly likely that transplant of anorgan derived from a living body causes immunological rejection, infectious diseases, or the like. And there are many examples of death due to those symptoms, after transplant. In addition, there are many patients who are desiring and waiting for transplant because of lack of donors. And the number of death of the waiting patients per year is larger than that of transplants. Even if the patients receive organ transplant, there are many problems in transplant of an organ derived from a living body, such as huge cost for transplant and prognostic treatment. In order to overcome those problems in organ transplant, regenerative medicine has attracted attention as a novel treatment method and has drawn a highest interest.

The regenerative medicine is a treatment method characterized in that tissues and organs are restructured by differentiating cells having pluripotency and self-replication ability for a lost or failed part in a human body. This treatment enables autograft performed by using cells of a patient himself, and is less likely to cause immunological rejection or infection. Moreover, the tissues and organs are formed using cells, so that the treatment is expected as a novel treatment method to solve lack of donors. Examples of known donor cells available for the regenerative medicine include an embryonic stem cell derived from a fertilized ovum (ES cell; Embryonic Stem Cell) and an adult stem cell derived from bone marrow stroma (MS cell; Marrow Stem Cell).

ES cells are undifferentiated cells derived from fertilized ova, and differentiation thereof into various tissues and organs can be induced. However, in order to establish the cells, it is necessary to use human fertilized ova, and there are ethical issues. On the other hand, MS cells, which are stem cells derived from bone marrow stroma, are considered to be useful for regeneration of bones, muscles, and fat tissues in the regenerative medicine. In addition, there are no ethical issues because the MS cells are somatic cells, and the cells can be collected from an adult body with relative ease. However, the bone marrow stroma contains various cells, so that it is difficult that only MS cells having pluripotency are isolated. Even if the MS cells are obtained, the rate of lost cells is high in proliferation culture, by due to incorporation of other cells. So there are many problems which should be solved in transplant treatment. Moreover, it is also a problem that donors are burdened too much because anesthesia is required for MS cells collection.

A great number of cells are required for transplant, so that it is essential to develop donor cells that may easily and inexpensively be supplied in large amounts for progress of regenerative medicine. Although stem cells have been studied intensively as donor cells for the regenerative medicine, supply, maintenance, and culture of those ES cells or MS cells require special reagents, equipments, and techniques and cost a great deal of money. For solving the problems, it is necessary to obtain cells that have pluripotency and self-replication ability, can be easily collected, and have characteristics that are stably maintained. And it is desirable to establish a method of acquiring cells to fulfill those conditions.

Accordingly, in order to solve those problems, the inventors of the present invention significantly departed from the conventional idea of donor cells for regenerative medicine, and focused on matured adipocytes existing in the body surface of each site of a living body. The "matured cells" means differentiated cells, and terminally differentiated cells are generally considered not to dedifferentiate. However, the inventors of the present invention have succeeded in establishment of a novel culture method of establishing preadipocyte cell lines by induction of dedifferentiation of matured adipocytes (JP-A-2000-83656). The preadipocyte cell lines established by the culture method are uniform, easily maintained and cultured, and special techniques and facilities, and the like are not required. Therefore, the cells are expected as novel donor cells for regenerative medicine to almost solve the problems in the stem cells.

The preadipocyte cell lines developed by the inventors of the present invention are derived from matured adipocytes existing near the body surface, in the form of subcutaneous fat tissue. The matured adipocytes can be easily collected as unitary cells containing no other cells and easily collected in large amounts in a situation which is less burdensome for donors. In addition, subcutaneous fats exist in from a neonate to a senior, so that donor cells can be obtained regardless of the age, and autograft can be performed. If immunological issues are solved, it is highly likely that the cells can be industrially mass-produced, by utilizing adipocytes which are discharged in esthetic surgery or the like. It is desirable in construction of autografting systems for regenerative medicine to establish a method of acquiring cells having other functions such as osteocytes, muscular cells, chondrocytes, epithelial cells, and neurocytes by using the dedifferentiation method of matured adipocytes into preadipocytes and the inducing transdifferentiation using the preadipocyte cell lines, which have been developed by the inventors of the present invention, and to form tissues and organs.

### [Disclosure of the Invention]

The present invention relates to a method of acquiring cells having other functions, by inducing transdifferentiation of preadipocytes obtained by dedifferentiating matured adipocytes derived from subcutaneous fat tissues.

Moreover, the present invention relates to application to regenerative medicine of tissues or organs, which are formed by using the cells acquired by the method.

That is, the present invention relates to a culture method of inducing transdifferentiation into other cell

The present invention provides a cell culture method comprising the steps:
a) obtaining a fraction of unilocular mature adipocytes derived from subcutaneous fat tissue sample obtained from a subject by filtration of the tissue sample, subjecting the fraction to centrifugation and recovering the unilocular adipocytes separated In the upper layer.
b) subjecting said mature adipocytes to ceiling culture and continuing the culture for at least a further 14 days with the cell adhered-surface at the bottom to obtain fibroblast-like preadipocytes having no lipid droplets, which act as preadiocytes expressing an early marker of osteogenesis, myogenesis or adipogenesis;
c) inducing transdifferentiation of said preadipocytes by culturing using differentiation-inducing medium to give a transdifferentiated cell selected from an osteoblast, a myoblast, a chondrocyte, a mammary epithelial cell and a neurocyte.

The present invention further provides inducing transdifferentiation, by culturing using differentiation-inducing medium, of the preadipacyte call line deposited as FERM BP-08645 to give a transdifferentiated cell selected from an osteoblast, a myoblast, a chondrocyte, a mammary epithelial cell and a neurocyte.

The transdifferentiated cell may be an osteoblast, a myoblast, a chondrocyte, a mammary epithelial cell or a neurocyte.

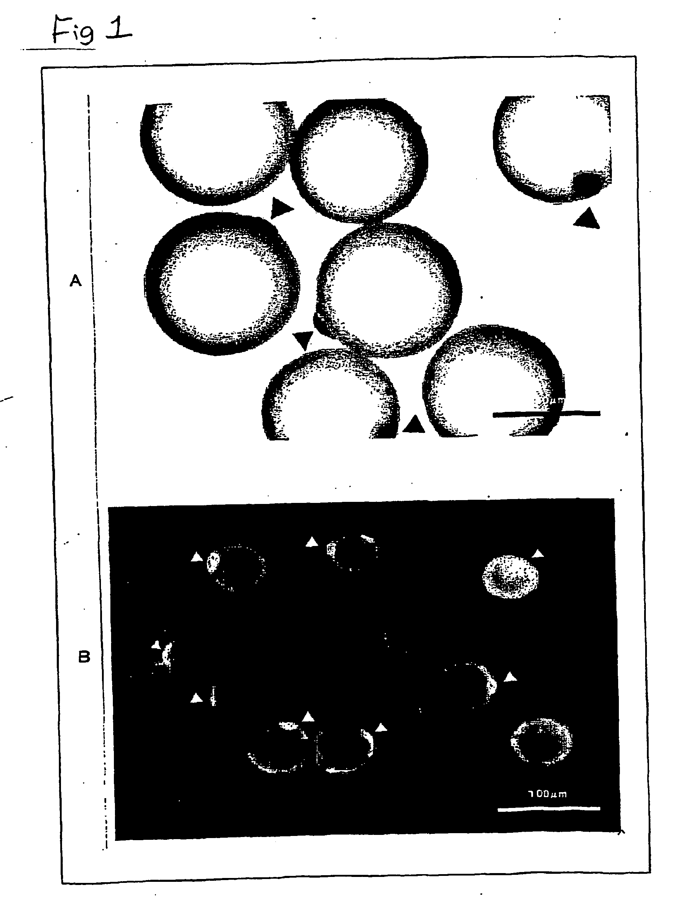

In the present invention, dedifferentiation of matured adipocytes derived from subcutaneous fat tissue may be carried out in accordance with JP-A-2000-83858 disclosed by the inventors of the present invention. That is, subcutaneous fat tissues are treated with collagenase, and filtration was performed with meshes (mesh size: 100 and 150 µm). So, a single fraction including only matured adipocytes was collected (Fig. 1) . Ceiling culture of those animal matured adipocytes is performed to form fibroblast-like adipocytes (Fibroblast-like Adipocytes: hereinafter, referred to as FAs). And the fibroblast-like adipocytes are subcultured for transdifferentiation, to thereby yield preadipocytes (Porcine Preadipocytes derived from Matured Adipocytes: PPMAs, hereinafter, referred to as PAs). Examples of such animal matured adipocytes include matured adipocytes derived from fat tissues of human, porcine, bovine, chicken, and the like. The matured adipocytes are cells derived from subcutaneous fat tissues.

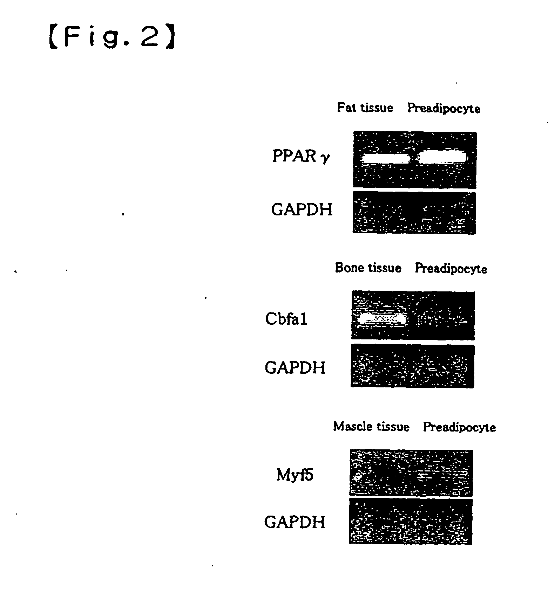

In a part of PAs dedifferentiated from matured adipocytes as described above, mRNA expression states of various transcription factors were investigated by the RT-PCR method. As a result, as shown in Fig. 2, the PAs were found to be hetero cells in which peroxisome proliferator-activated receptor γ2 (hereinafter, abbreviated to PPARγ2, the upper photograph) relating to commitment in the early process of adipocyte differentiation in the growth phase, Cbfa1 the middle photograph) relating to determination in the differentiation process of osteogenesis, and Myf5 (the lower photograph) relating to determination in the differentiation process of myogenesis have already been expressed. That is, the PAs are hetero cells in which early markers of osteogenesis, myogenesis, or adipognesis have already been expressed, and they are in a "wobble" state and unique cells different from stem cells. A PA line derived from porcine-matured adipocytes having such characteristics is internationally deposited to International Patent Organism Depositary, National Institute of Advanced Industrial Science and Technology (address: Central 6, Higashi 1-1-1, Tsukuba, Ibaraki, Japan) under Budapest Treaty, and deposit No. FERM BP-08645 is assigned (deposit date: February 20, 2004).

### [Brief Description of the Drawings]

Fig. 1 shows micrographs of porcine-matured adipocytes and mouse-matured adipocytes isolated from fat tissues.
   A; Nuclei of porcine-matured adipocytes were stained by hematoxylin staining.
   B; This figure shows a fluorescent microscope image of matured adipocytes derived from a GFP mouse. Nuclei and cytoplasms are fluorescing.

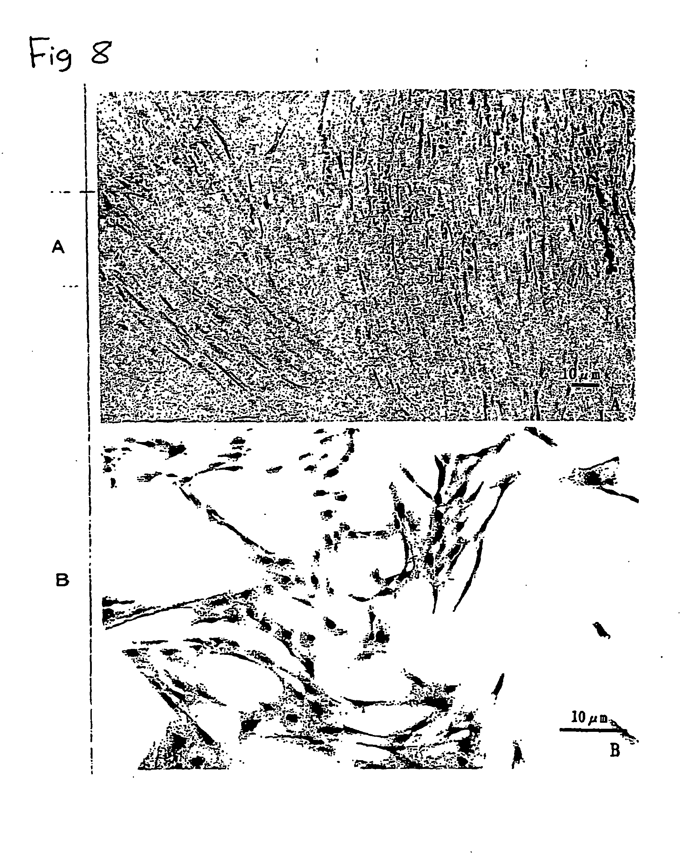

Fig. 2 shows photographs of expression states of various transcription factors of preadipocytes derived from mouse-matured adipocytes in the growth phase. The photographs show PPARγ2 (upper), Cbfa1 (middle), and Myf5 (lower) expressions.
Fig. 3 shows micrographs of osteoblasts obtained by transdifferentiation of PAs derived from matured adipocytes in Examples.
   A. This figure shows alkaline phosphatase-positive osteoblasts after 28 days of induction of differentiation.
   B. This figure shows shapes of alkaline phosphatase-positive cells in high magnification. The alkaline phosphatase-positive cells have shapes unique to osteoblasts.
Fig. 4 shows micrographs of osteoblasts obtained by transdifferentiation of PAs derived from matured adipocytes in Examples.
   A. Alkaline phosphatase-positive osteoblasts which derived from a mouse fetal cranial bone, after 4 days of induction of differentiation (control).
   B. This figure shows osteoblasts (blue) derived from matured adipocytes after 14 days of induction of differentiation. There are both osteoblasts (blue) and adipocytes (red).

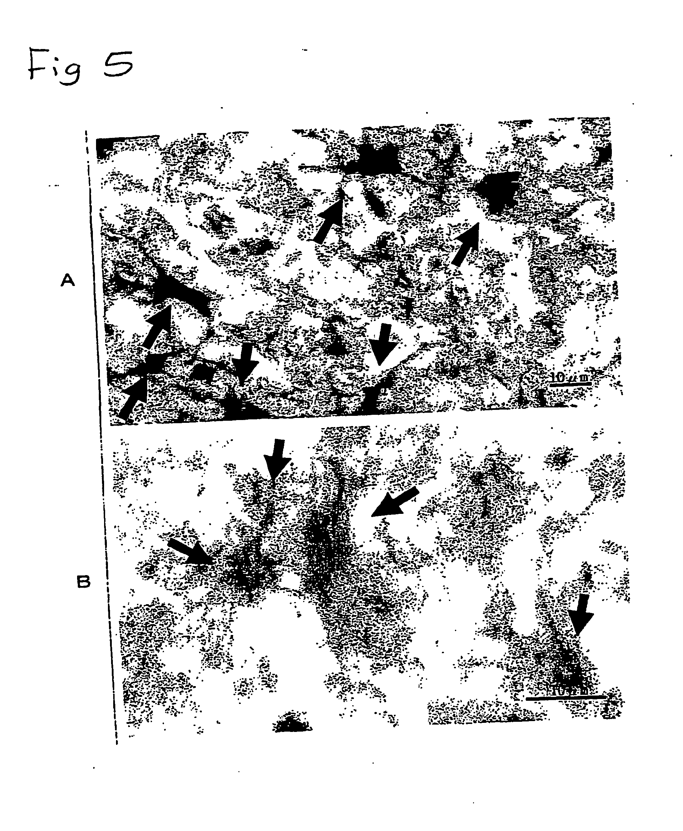

Fig. 5 shows micrographs of osteoblasts that are derived from matured adipocytes and secrete osteocalcin in Examples.
   The arrows show osteocalcin antibodies and stained osteoblasts derived from matured adipocytes after 24 days of induction of differentiation into osteoblasts.

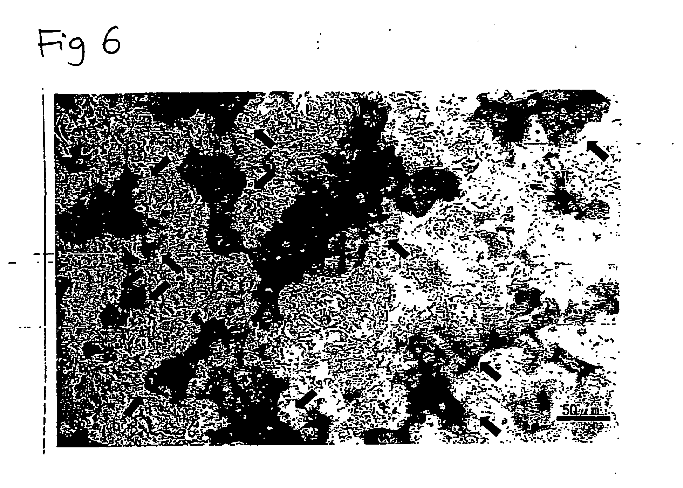

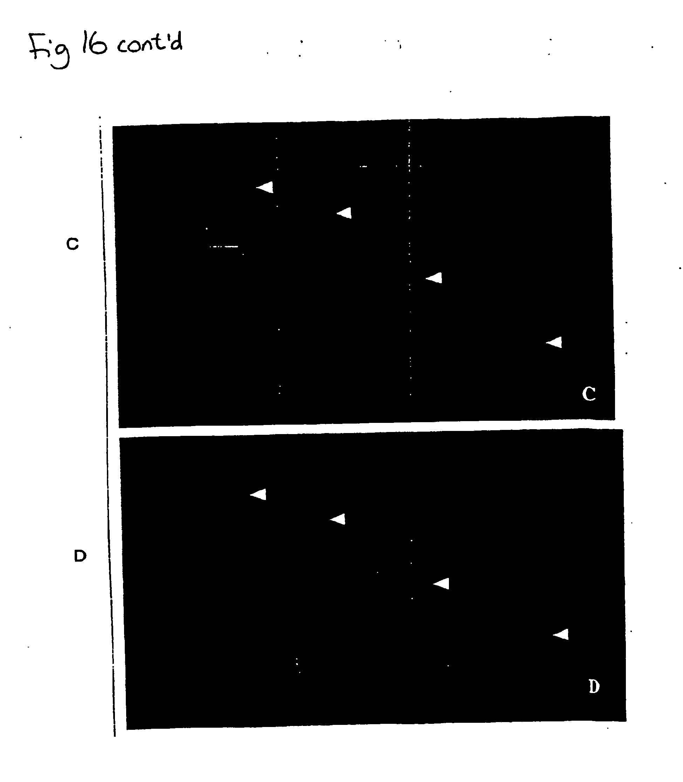

Fig. 6 shows a micrograph of formation of bone matrices (calcium deposition) of osteoblasts derived from matured adipocytes in Examples.
   The arrows show that calcium deposition portions subjected to Kossa-staining after 20 days of induction of differentiation are stained.

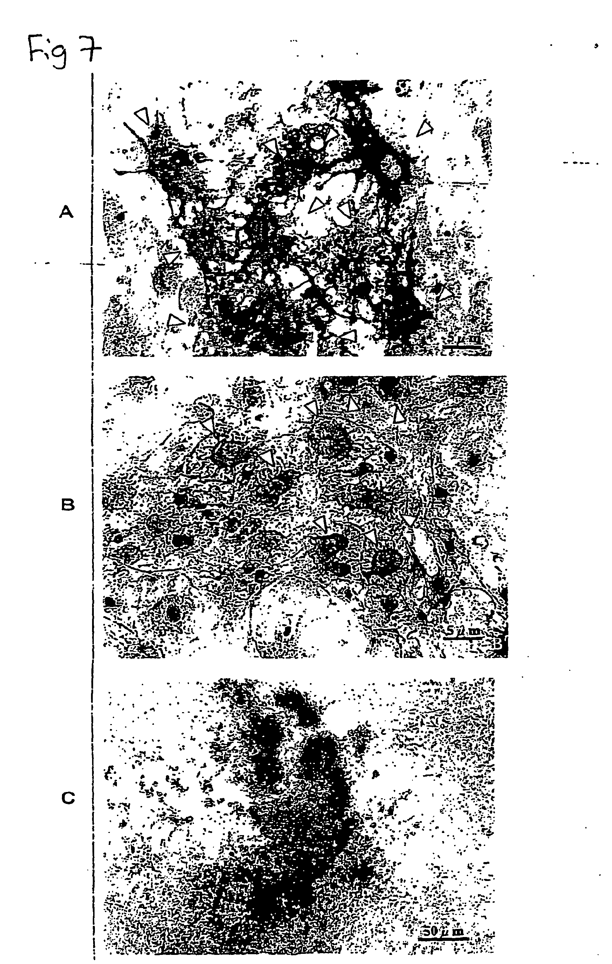

Fig. 7 show micrographs of osteoblasts obtained by transdifferentiation of PAs derived from mouse-matured adipocytes in Examples.
   A. This figure shows alkaline phosphatase-positive osteoblasts after 28 days of induction of differentiation.
   B. This figure shows a micrograph of osteoblasts that are derived from matured adipocytes and secrete osteopontin in Examples. The arrows show osteocalcin antibodies and stained osteoblasts derived from matured adipocytes after 12 days of induction of differentiation into osteoblasts.
   C. This figure shows a micrograph of formation of bone matrices (calcium deposition) of osteoblasts derived from matured adipocytes in Examples. Calcium deposition portions subjected to Kossa-staining after 16 days of induction of differentiation are stained
Fig. 8 shows micrographs of myoblasts obtained by transdifferentiation of PAs derived from matured adipocytes in Examples.
   A. Control. Myf5 is not expressed in nuclei.
   B. Myf5 expressions are observed in nuclei of most cells after 4 days of induction of differentiation.

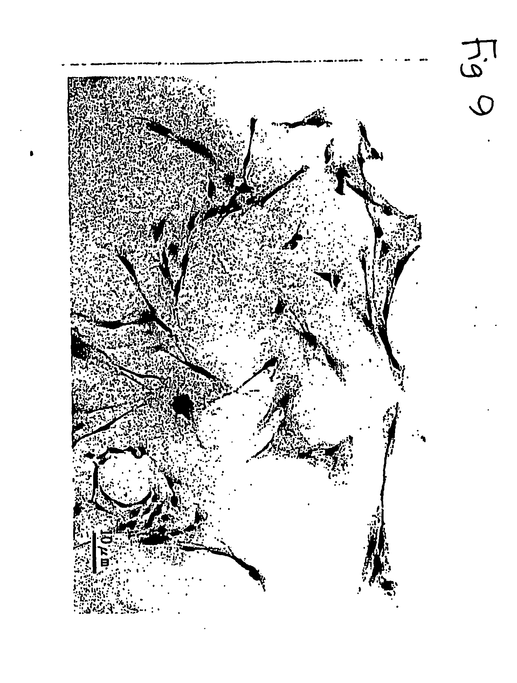

Fig. 9 shows a micrograph of myoblasts obtained by transdifferentiation of PAs derived from matured adipocytes in Examples.
   MyoD expressions are observed in nuclei of most cells after 6 days of induction of differentiation.

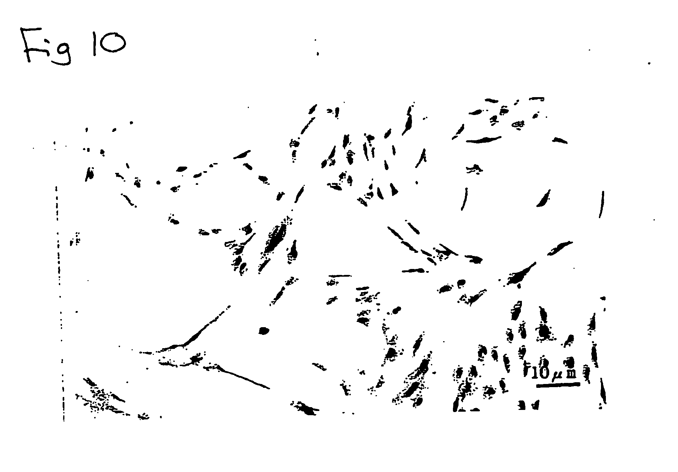

Fig. 10 shows a micrograph of myoblasts derived from matured adipocytes, in which myogenin is expressed in Examples.
   Myogenin expressions are observed in nuclei of most cells after 18 days of induction of differentiation.

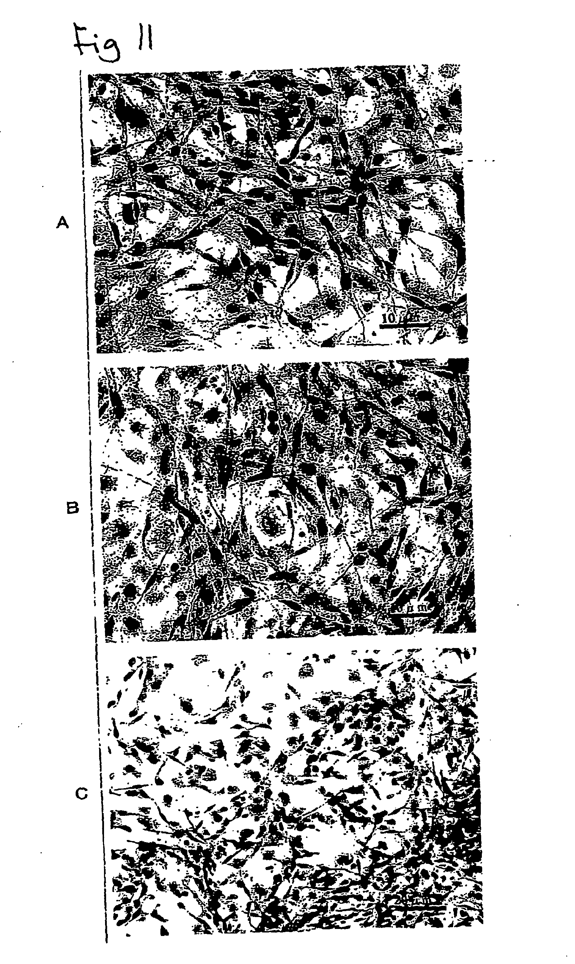

Fig. 11 shows micrographs of myoblasts obtained by transdifferentiation of PAs derived from mouse-matured adipocytes in Examples.
   A. Myf5 expressions are observed in nuclei of most cells after 4 days of induction of differentiation.
   B. MyoD expressions are observed in nuclei of most cells after 4 days of induction of differentiation.
   C. Myogenin expressions are observed in nuclei of most cells after 7 days of induction of differentiation.

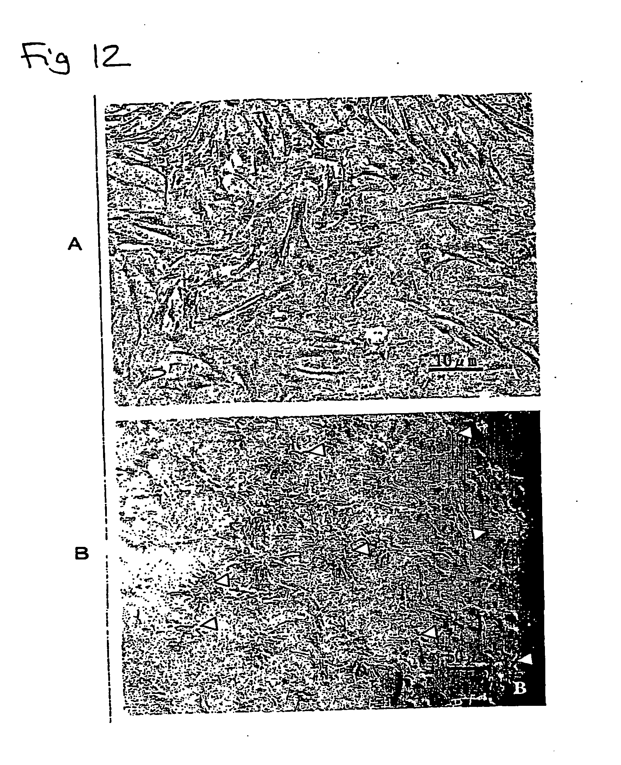

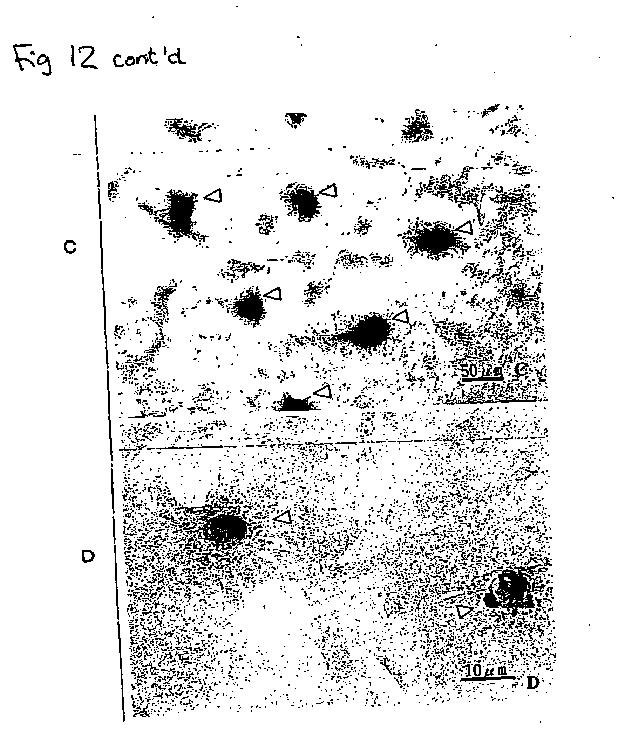

Fig. 12 shows micrographs of chondrocytes obtained by transdifferentiation of PAs derived from matured adipocytes in Examples.
   A. This figure shows control PAs after 16 days of culture. There is observed no cell-aggregate to be observed in the case of induction of transdifferentiation into chodrocytes. The control cells were not both stained by alcian blue staining and toluidine blue staining, not immunostaining with collagen type 2 (data not shown).
   B. This figure shows an alcian blue staining image after 16 days of induction of differentiation. The cells formed aggregates, and the interiors thereof were stained (see the arrow heads).
   C. This figure shows a toluidine blue staining image after 16 days of inductionof differentiation. The cells formed aggregates, and the interiors thereof were stained (see the arrow heads).
   D. This figure shows an immunostaining image with collagen type 2 after 16 days of induction of differentiation. The cells formed aggregates, and the interiors thereof were stained (see the arrow heads).

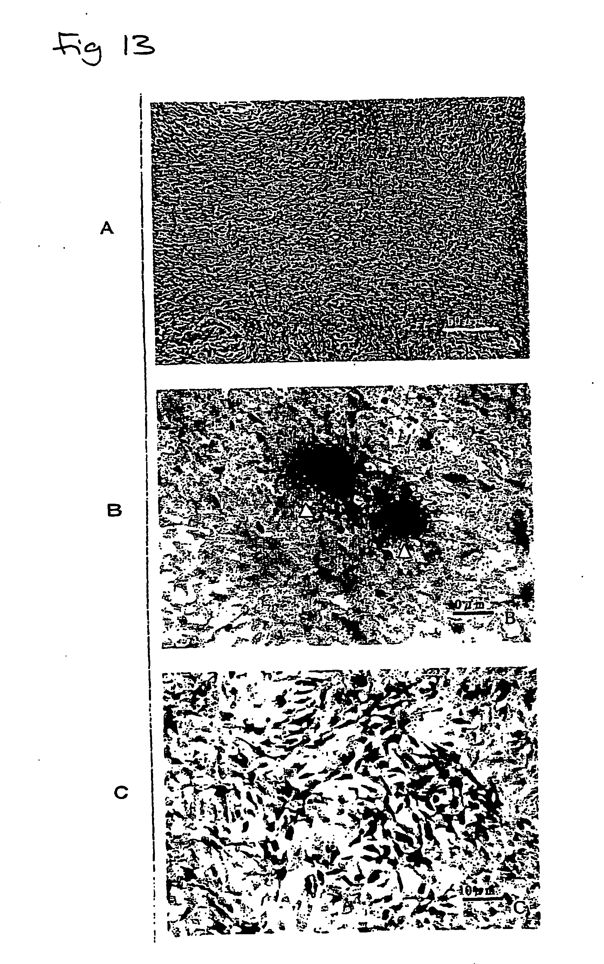

Fig. 13 shows micrographs of chondrocytes obtained by transdifferentiation of PAs derived from mouse-matured adipocytes in Examples.
   A. This figure shows an alcian blue staining image after 14 days of induction of differentiation. Most cells were stained.
   B. This figure shows a toluidine blue staining image after 14 days of induction of differentiation. Most cells were stained. In some portions, aggregates were formed (see the arrow heads).
   C. This figure shows an immunostaining image with collagen type 2 after 14 days of induction of differentiation.

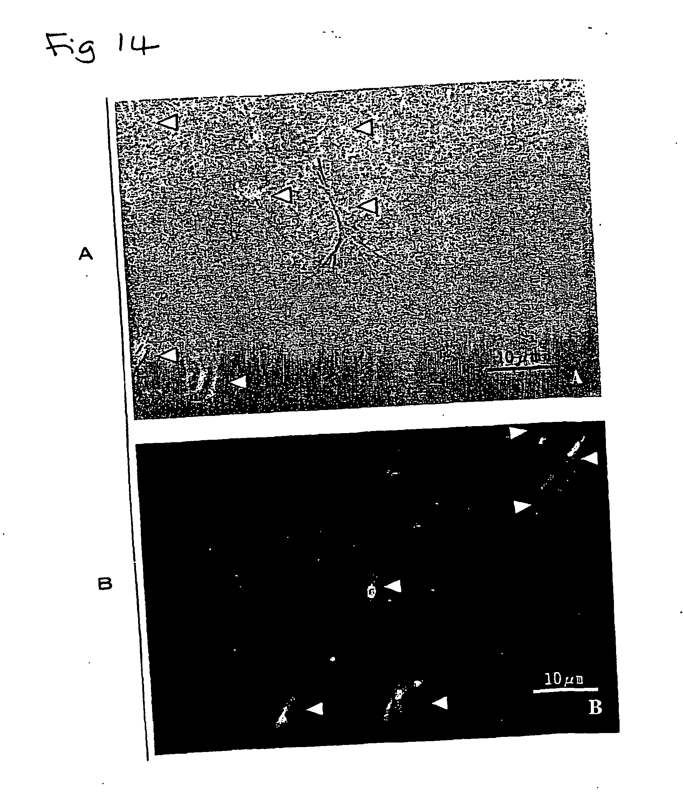

Fig. 14 shows micrographs of mammary epithelial cells (MEs) obtained by transdifferentiation of PAs (GFP-PAs) derived from matured adipocytes in Examples.
   A. This figure shows an optical microscope image of GFP-PAs and MEs (arrow heads) after 2 days of the three-dimensional culture with a collagen gel. In the three-dimensional culture, both GFP-PAs and MEs have fibroblast-like shapes.
   B. This figure shows an optical microscope image of GFP-PAs and MEs (arrow heads) after 2 days of three-dimensional culture with a collagen gel. It was confirmed that the fibroblasts having nuclei that strongly fluoresced green under ultraviolet radiation are GFP-PAs. The wild-type MEs do not fluoresce.

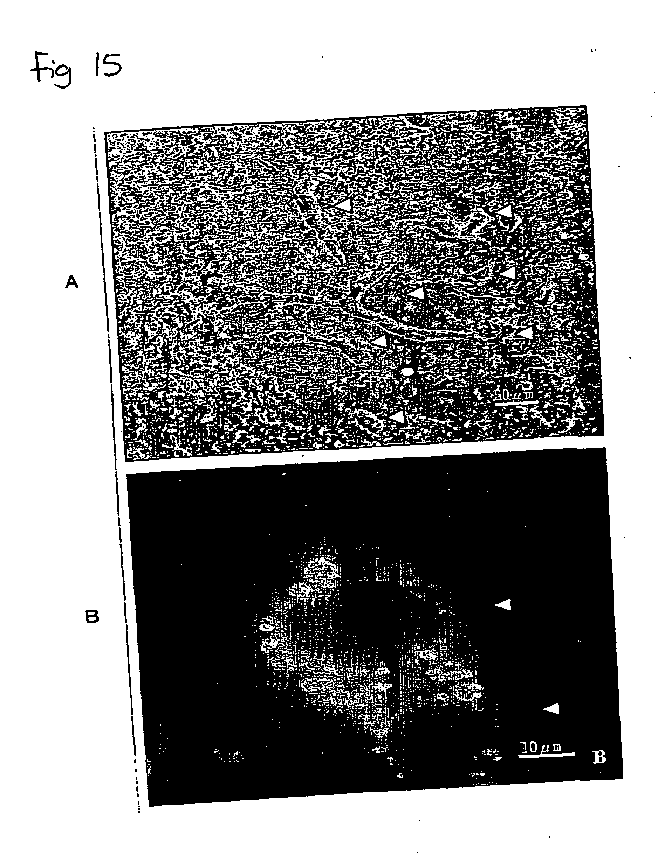

Fig. 15 shows micrographs of mammary epithelial cells (ME) obtained by transdifferentiation of PAs (GFP-PAs) derived from matured adipocytes in Examples.
   A. After 28 days of induction of differentiation, the cells assembled to have mammary duct-like shapes (arrow heads).
   B. The cells that fluoresced under a fluorescent microscope and had mammary duct-like shapes after 28 days of induction of differentiation are derived from GFP-PA, and the shapes varied into epithelial cell-like shapes to form alveolar structures.

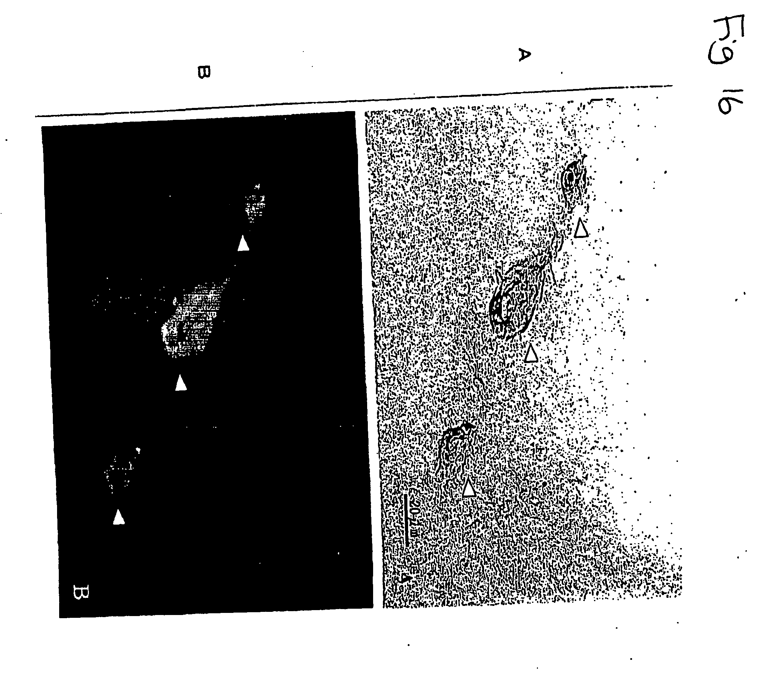

Fig. 16 shows micrographs of mammary epithelial cells (MEs) obtained by transdifferentiation of PAs (GFP-PAs) derived from matured adipocytes in Examples.
   A. This figure shows an optical microscope image of GFP-PAs and MEs (arrow heads) stained with hematoxylin after 28 days of induction of differentiation. The cells in collagen gel assembled to form alveolar structures.
   B. This figure shows a fluorescent microscope image of the cells forming alveolar structures in A. The nuclei of the cells forming alveolar structures fluoresced strongly, so that it was confirmed that those epithelial cell-like cells are derived from GFP-PAs.
   C. This figure shows an E-cadherin immunostaining image in the same specimen as that in B. Unlike the case of B, it was confirmed that whole cells forming alveolar structures fluoresced (arrow heads).
   D. This figure shows a keratin immunostaining image. Unlike the case of B, whole cells forming alveolar structures fluoresced (arrow heads).

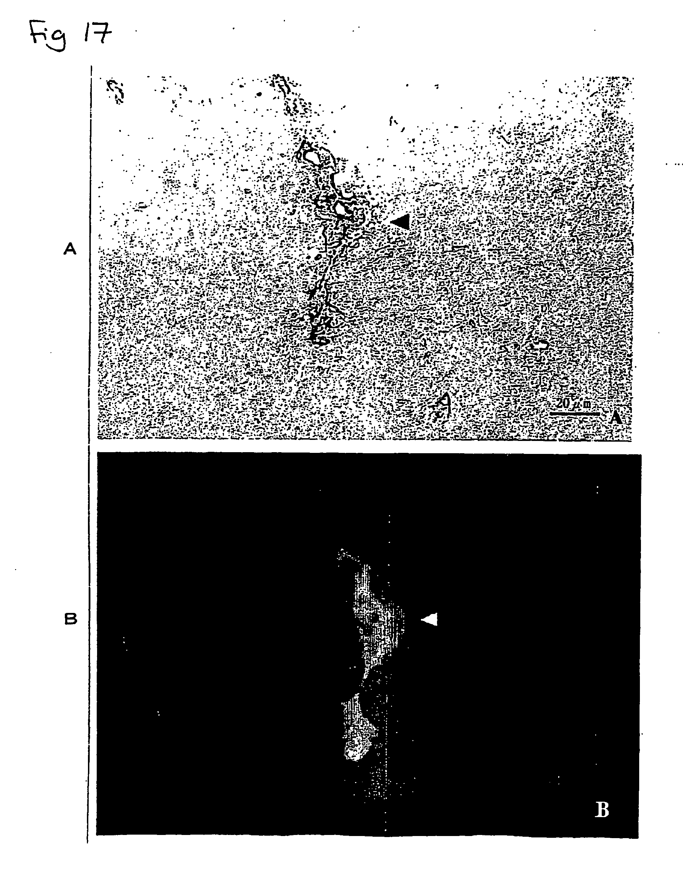

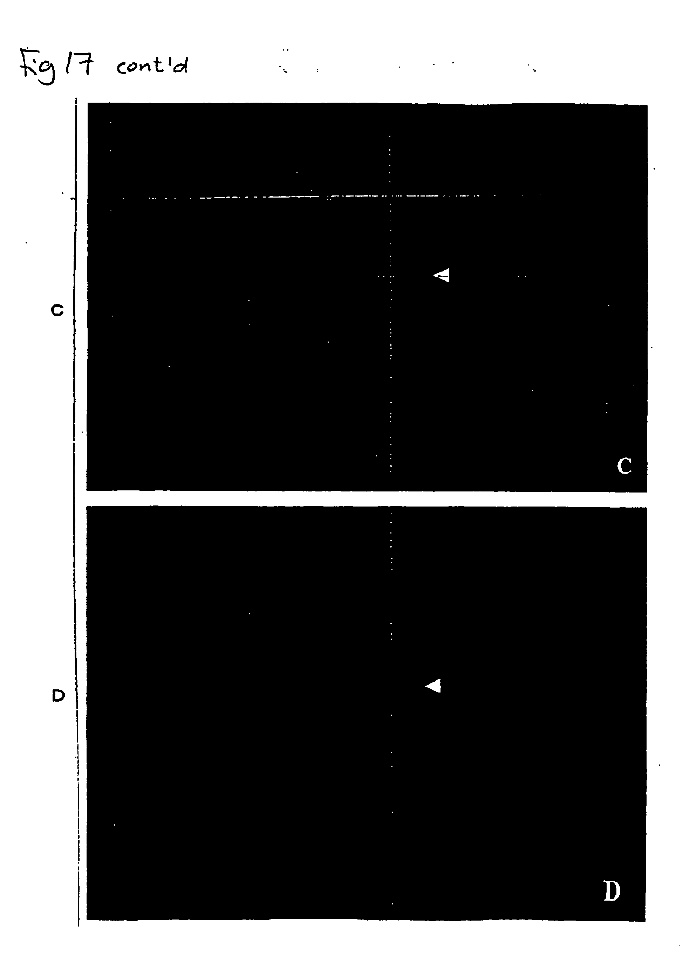

Fig. 17 shows micrographs of mammary epithelial cells (MEs) obtained by transdifferentiation of PAs (GFP-PAs) derived from matured adipocytes in Examples.
   A. This figure shows an optical microscope image of GFP-PAs and MEs (arrow heads) stained with hematoxylin after 28 days of induction of differentiation. The cells in the collagen gel assembled to form tubular structures.
   B. This figure shows a fluorescent microscope image of cells that formed alveolar structures in A. The cells having tubular structures fluoresced strongly, so that it was confirmed that those epithelial cell-like cells are derived from GFP-PAs.
   C. This figure shows a vinculin immunostaining image of the same specimen as that in B. It was confirmed that whole cells forming tubular structures fluoresced (arrow head).
   D. This figure shows a ZO-1 immunostaining image. It was confirmed that whole cells forming tubular structures fluoresced (arrow head).

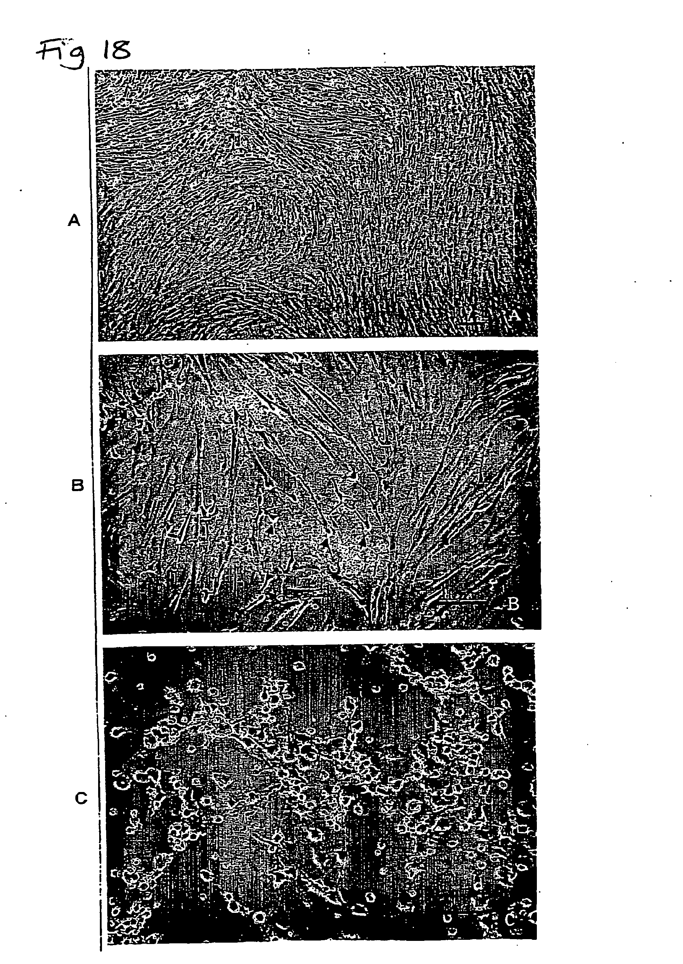

Fig. 18 shows micrographs of neurocytes obtained by transdifferentiation of PAs derived from matured adipocytes in Examples.
   A. This figure shows PAs in which differentiation into neurocytes had not been induced. All the cells maintained the fibroblast-like shapes.
   B. This figure shows an optical microscope image of PAs after 12 hours of induction of differentiation. There are observed fibroblast-like PAs and cells having shapes that were varied into neurocyte-like shapes (arrow heads).
   C. This figure shows Ng108-15 cell line as a control. After induction of differentiation, Ng108-15 showed a shape unique to a neurocyte.

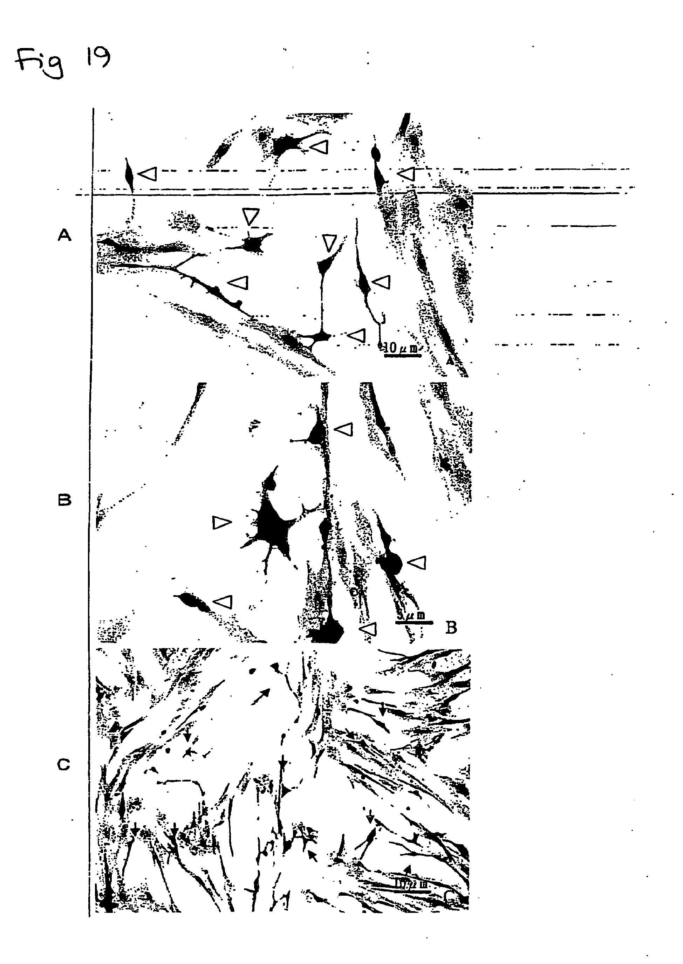

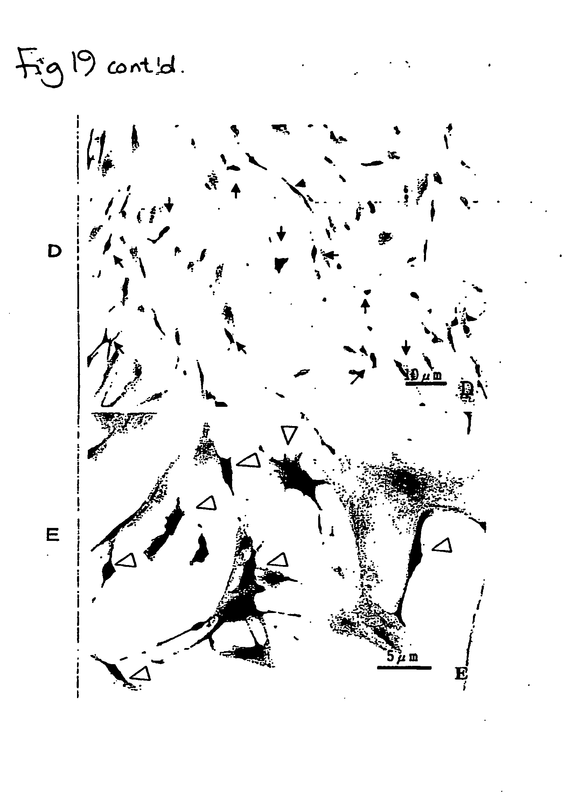

Fig. 19 shows micrographs of neurocytes obtained by transdifferentiation of PAs derived from matured adipocytes in Examples.
   A. After 17 hours of induction of differentiation, only cells having neurocyte-like shapes were stained with a nestin antibody (arrow heads) .
   B. After 17 hours of induction of differentiation, only cells having neurocyte-like shapes were stained with a neuron-specific enolase antibody (arrow heads).
   C. After 17 hours of induction of differentiation, only cells having neurocyte-like shapes were stained with a BIII-tubulin antibody.
   D. After 17 hours of induction of differentiation, only cells having neurocyte-like shapes were stained with an MAP2 antibody.
   E. After 17 hours of induction of differentiation, only cells having neurocyte-like shapes were stained with a neurofilament antibody (arrow heads).

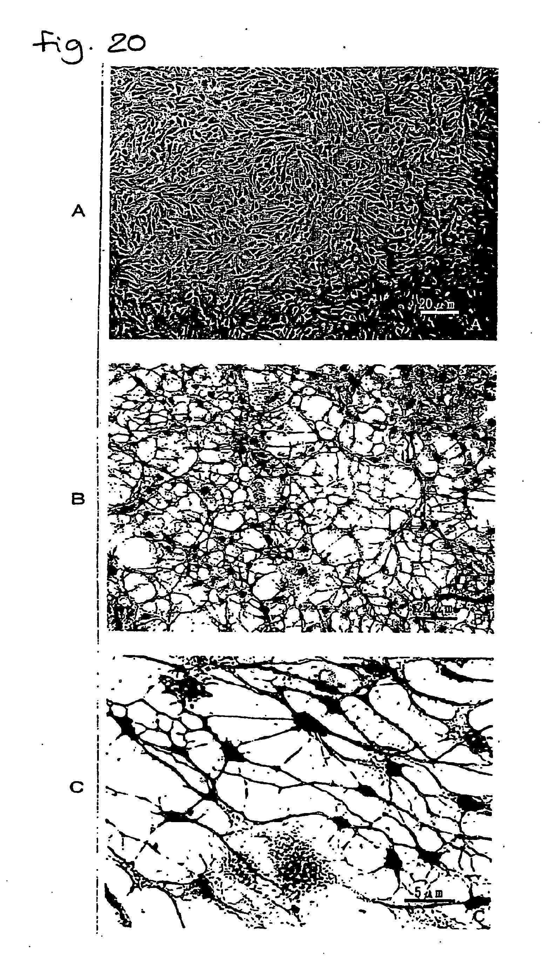

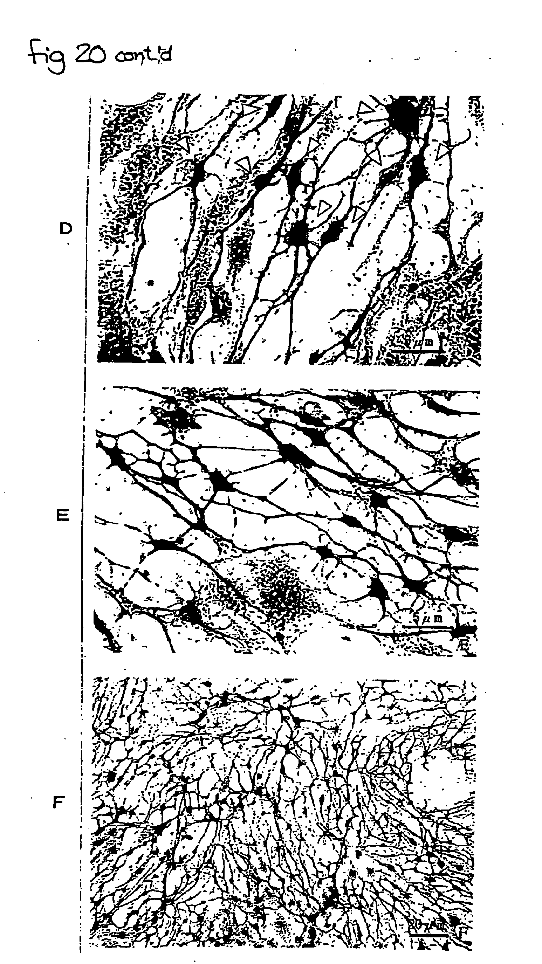

Fig. 20 shows micrographs of neurocytes obtained by transdifferentiation of PAs derived from mouse-matured adipocytes in Examples.
   A. This figure shows PAs in which differentiation into neurocytes had not been induced. All the cells maintain fibroblast-like shapes.
   B. After 17 hours of induction of differentiation, only cells having neurocyte-like shapes were stained with a nestin antibody.
   C. After 17 hours of induction of differentiation, only cells having neurocyte-like shapes were stained with a neuron-specific enolase antibody.
   D. After 17 hours of induction of differentiation, only cells having neurocyte-like shapes were stained with a BIII-tubulin antibody.
   E. After 17 hours of induction of differentiation, only cells having neurocyte-like shapes were stained with an MAP2 antibody.
   F. After 17 hours of induction of differentiation, only cells having neurocyte-like shapes were stained with a neurofilament antibody.

### [Best Mode for carrying out the Invention]

Hereinafter, the present invention will be described in detail.

In the present invention, osteoblasts, myoblasts, chondrocytes, epithelial cells, or neurocytes are acquired by induction of transdifferentiation of PAs derived from porcine-matured adipocytes and PAs derived from mouse-matured adipocytes. As the transdifferentiation method, any of the conventional methods to be used for transdifferentiation of cells may be used. In particular, the following procedure is preferable: the PA line is suspended in a medium supplemented with serum; the suspension is inoculated in a tissue-culture dish or flask to which collagen type 1 or type 3 has been applied; the cells are cultured under humidified atmosphere of 5% CO₂ and 95 % air; the medium is exchanged for a differentiation-inducing medium at the time of achieving confluent growth; and the cells are cultured for 10 to 20 days.

As the differentiation-inducing medium, any medium to be used as conventional differentiation-inducing mediums may be used. For example, osteoblasts are preferably cultured for 10 to 20 days, in a Dulbecco's modified Eagle's medium supplemented with active vitamin D₃, ascorbic acid, β-glycerophosphoric acid, and serum, or dexamethasone. Myoblasts are preferably cultured for 10 to 18 days in a Dulbecco's modified Eagle's medium supplemented with hydrocortisone and serum, while chondrocytes are preferably cultured for 2 weeks in Dulbecco' s modified Eagle' s medium supplemented with insulin, ascorbic acid, transforming growth factor β3, and serum. Meanwhile, epithelial cells are preferably cultured for 10 to 18 days in Dulbecco's modified Eagle's medium supplemented with prolactin, dexamethasone, ITS (insulin-transferrin-selenium), Hepes (N-(2-Hydroxyethyl)piperazine-N'-(2-ethanesulfonic acid)), and serum, while neurocytes are preferably cultured for 12 hours in Dulbecco's modified Eagle's medium supplemented with β-mercaptoethanol and serum, and then cultured for 5 hours in Dulbecco's modified Eagle's medium supplemented with β-mercaptoethanol.

For identifying osteoblasts obtained by transdifferentiation of the thus-cultured cells, the following are preferably performed as indices: alkaline phosphatase staining and determination of specific activity value; immunostaining with an osteocalcin antibody; and a von Kossa histochemical method and formation of a calcified extracellular matrix.

For separating osteoblasts from the medium, the cells are firstly liberated from the medium and suspended in a culture medium, and the suspension is centrifuged to separate adipocytes in which lipid droplets are accumulated in the upper layer and osteoblasts in the lower layer (precipitant fraction), followed by collection of the osteoblasts in the lower layer.

Identification of myoblasts are preferably performed by immunostaining with Myf5, MyoD, and a myogenin antibody, which are muscle determination factors, as indices, while for identifying chondrocytes, alcian blue staining, toluidine blue staining, and immunostaining with a collagen type 2 antibody are preferably performed as indices. For identifying epithelial cells, immunostaining with E-cadherin, vinculin, keratin, and ZO-1 antibodies are preferably performed as indices, while for identifying neurocytes, immunostaining with nestin, neuron-specific enolase, βIII-tubulin, MAP2, and a neurofilament antibody are preferably performed as indices.

### [Examples]

Hereinafter, specific examples of the present invention will be represented, but the present invention should not be construed as limited thereto in any case.

### (1) Induction of transdifferentiation of matured adipocyte into osteoblast

PA line derived form matured adipocytes were produced using matured adipocytes in porcine subcutaneous fat tissues and mouse subcutaneous fat tissues by the method described in JP-A-2000-83656.

That is, a porcine PA line was obtained by the following steps: 4 g of a subcutaneous fat tissue collected from a 6-month old male porcine was put in a Dulbecco's modified Eagle' s medium containing Hepes (Hepes-DMEM; Nissui Pharmaceutical Co., Ltd.) supplemented with 1% collagenase (type II; SIGMA) to treat it with collagenase, and then filtration was performed using a nylon mesh, to thereby yield a cell suspension. The resultant cell suspension was centrifuged for 3 minutes at 106 G, and a matured adipose fraction separated in the upper layer was added to a fresh Hepes-DMEM medium supplemented with 3% FCS. Then, centrifugation for 3 minutes at 106 G was repeated three times, to thereby yield matured adipocytes. The matured adipocytes were transferred to a tissue culture flask (Falcon, 3107), and the flask was fully filled with a DMEM supplemented with 20% FCS, 1.8 mg/ml NaHCO₃, and 0.08 mg/ml kanamycin sulfate. Then, the flask was allowed to stand so that the bottom of the flask was turned up in an incubator at 37 °C under humidified atmosphere of 5% CO₂ and 95 % air, followed by culture for 6 days. After 4 days of culture, most of cells were firmly adhered to the ceiling plane of the flask, and shapes of the cells were converted to shapes of multilocular adipocytes having various sizes of lipid droplets around a large lipid droplet. After 6 days of culture, the lipid droplets became smaller, and many cells having shapes converted to fibroblast-like (FA) shapes having no lipid droplets were observed. After 6 days of culture, the medium in the flask was exchanged for a DMEM supplemented with 20% FCS, and culture was continued for 16 days so that the cell adhered-surface was the bottom in the CO₂ incubator. The medium was exchanged every 4 days. FAs having no lipid droplets were actively proliferated, and after 14 days of culture, all cells in the flask became FAs and achieved confluent growth.

FAs derived from porcine-matured adipocytes have active proliferation potency, and has differentiation potency for redifferentiating into adipocytes having lipid droplets by a differentiation inducer such as DEX, INS, or IBMX, so that they were produced as PAs derived from matured adipocytes (Fig. 1A). Hereinafter, PAs to be used in Examples were produced in the same manner as above.

The resultant PAs were resuspended to 1 × 10⁴ cells/ml in a DMEM supplemented with 20% serum. Thereafter, the suspension was seeded into a culture dish (Falcon, 3001) for tissue culture on which collagen type 1 or 3 had been coated, and the dish was allowed to stand to culture the cells in an incubator at 37 °C under humidified atmosphere of 5% CO₂ and 95 % air.
Note that, the medium was exchanged every 4 days. After 8 days of culture, the medium including confluent PAs was exchanged for a DMEM supplemented with 0.1 µM dexamethasone and 10% serum (differentiation-inducing medium), followed by culture for 10 days.

Meanwhile, a mouse PA line was obtained by the following steps: 2g of a subcutaneous fat tissue was collected from a 6-week old male mouse, which is a transgenic mouse to which a green fluorescent protein (GFP) gene had been introduced. And a cell suspension was obtained by the same method as above.

The resultant cell suspension was used in the same manner as above to yield matured adipocytes, and the cells were cultured in the same manner as above. At that stage where many cells having shapes converted to fibroblast-like (FA) shapes having no lipid droplets were observed, the culture was continued so that the cell adhered-surface become the bottom in the same manner as above, to thereby yield FAs that have no lipid droplets and proliferate actively.

The FAs derived from the mouse-matured adipocytes have active proliferation potency and have differentiation potency for redifferentiating into adipocytes having lipid droplets by a differentiation inducer such as DEX, INS, or IBMX, so that FAs were producedasaPAline (GFP-PA) which is derived from matured adipocytes (Fig. 1B). Hereinafter, PAs to be used in Examples were produced in the same manner as above.

The produced PAs were cultured by the same method as above, and after 8 days of culture, the medium including confluent PAs was exchanged for a DMEM supplemented with 0.1µM dexamethasone and 10% serum (differentiation-inducing medium), followed by culture for 10 days. Meanwhile, expression statuses of various transcriptional factors in the growth phase were investigated, and the expressions of PPARγ, Cbfa1, and Myf5 were shown in Fig. 2.

### (2) Method of identifying osteoblast

The following methods identified that PAs derived from porcine-matured adipocytes and PAs derived from mouse-matured adipocytes produced by the method (1) above are respectively differentiated into osteoblasts. That is, in order to identify osteoblasts, the following were performed as indices: alkaline phosphatase staining and determination of specific activity value; immunostaining with an osteocalcin antibody; and the von Kossa histochemical method and formation of a calcified extracellular matrix.

### 1) Alkaline phosphatase (AP) and oil red (OR) O staining

PAs derived from porcine-matured adipocytes and PAs derived from mouse-matured adipocytes were fixed by the following method after 8 days and 7 days of induction of differentiation, respectively, and double staining was performed with alkaline phosphatase (AP) and oil red (OR) O. Then, 1 ml of a 4% formalin solution was added to a differentiation-inducing medium in a culture dish, and the dish was allowed to stand at room temperature for 20 minutes for prefixation. After removing the prefixative solution, 2 ml of a 4% formalin solution was further added thereto, and the dish was allowed to stand at room temperature for 1 hour. After removing the fixative solution, washing was performed with 2 ml of distilled water three times. Then, 0.5 ml of n-n dimethylformamide (Wako Pure Chemical Industries, Ltd.) to which 8 mg of naphthol AS-TR phosphate Na (SIGMA) had previously been added was mixed in 50 ml of a 0.1 M Tris buffer solution (Tris-HCl pH 9.0) in which 40 mg of Fast Blue BB (Wako Pure Chemical Industries, Ltd.) had been dissolved. Then, MgCl₂ was further added thereto, and the mixture was filtrated to prepare an AP staining solution. Subsequently, 2 ml of the prepared AP staining solution was added thereto, and the mixture was allowed to stand in an incubator at 37°C for 1 hour. After removing the AP staining solution, washing was performed with 2 ml of distilled water three times. Thereafter, 100 ml of isopropyl alcohol supplemented with 0.5 g of ORO (SIGMA) was mixed with distilled water at a ratio of 3:2, followed by filtration. Then, 2 ml of the ORO staining solution was added thereto, and the mixture was allowed to stand at room temperature for 20 minutes.

### 2) Imunostaining with osteocalcin antibody

PAs derived from porcine-matured adipocytes and PAs derived from mouse-adipocytes were fixed by the same fixation method as above after 16 days and 12 days of induction of differentiation, respectively, and washing was performed with phosphate buffer saline (PBS). Washing was performed with a 2% hydrogen peroxide solution in PBS three times to inhibit an intrinsic peroxidase activity. After intrinsic avidin-biotin had been inhibited, blocking was performed with PBS supplemented with normal serum for 20 minutes, and an osteocalcin antibody (diluted 400-fold) was allowed to react at 4°C for 20 hours. After washing the cells with PBS twice, a diluted biotinylated secondary antibody was allowed to react for 30 minutes, and washing was performed with PBS twice. Subsequently, ABC reagent was allowed to react for 60 minutes. After completion of the reaction, washing was performed with Tris-HCl, and DAB staining was performed for 10 minutes. Washing was performed with distilled water three times, followed by observation.

### 3) von Kossa histochemical methods

PAs derived from porcine-matured adipocytes and PAs derived from mouse-matured adipocytes were separately fixed by the same fixationmethod as above after 16 days of induction of differentiation, and washing was performed with phosphate buffer saline (PBS) three times. The dish was immersed in 5% silver nitrate in PBS for 60 minutes with exposure to ultraviolet radiation. Washing was carefully performed with distilled water three times, and the dish was immersed in a 5% sodium thiosulfate solution for three minutes. Washing was performed with distilled water twice, followed by observation.

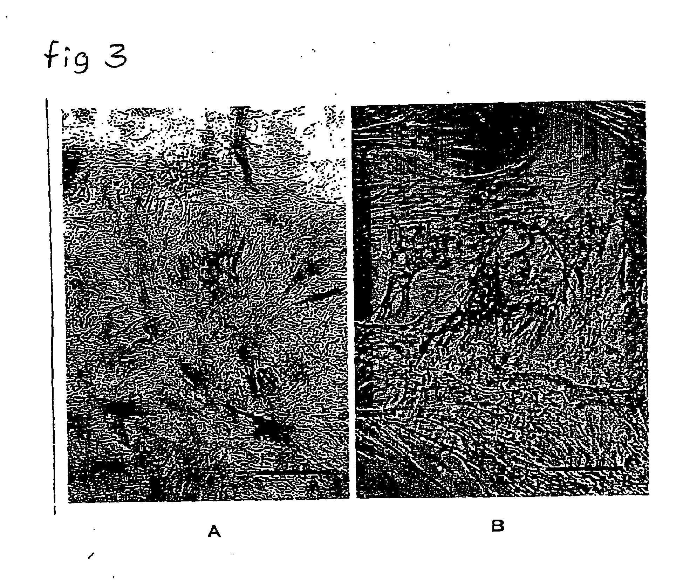

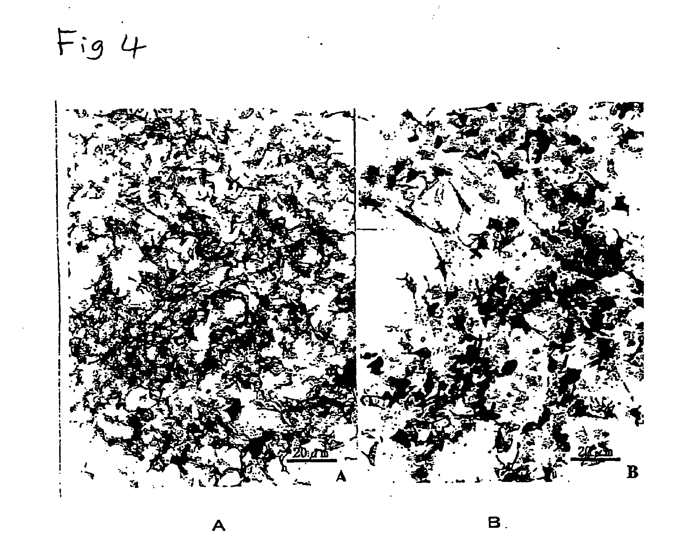

Figs. 3 to 6 show micrographs of the osteoblasts obtained by induction of differentiation of PAs derived from porcine-matured adipocytes. Meanwhile, Fig. 7 shows micrographs of osteoblasts obtained by induction of differentiation of PAs derived from mouse-matured adipocytes. As is clear from those figures, it was confirmed that osteoblasts can be acquired by induction of transdifferentiation of PAs derivedfrom porcine-matured adipocytes and PAs derived from mouse-matured adipocytes.

### (3) Method of separating osteoblast and adipocyte

Cells were washed with PBS containing no calcium and magnesium three times, and the cells were treated with PBS supplemented with 0.1% trypsin and 0.01% EDTA for three minutes. After confirming that those cells were completely liberated, a DMEM supplemented with 20% bovine FCS was added to suspend the cells. The cells were transferred to a centrifugation tube, and centrifugation was performed at 800 G to separate adipocytes in which lipid droplets were accumulated in the upper layer and osteoblasts in the precipitate fraction. The adipocytes in the upper layer were removed to acquire the osteoblasts in the precipitate fraction.

### (4) Induction of transdifferentiation of matured adipocyte into myoblast

PAs derived from porcine-matured adipocytes and PAs derived from mouse-matured adipocytes produced by the method described in (1) above were separately resuspended to 1 × 10⁴ cells/ml in a DMEM supplemented with 20% serum. Thereafter, each suspension was inoculated in a culture dish (Falcon, 3001) for tissue culture on which collagen type 1 had been coated, and the dish was allowed to stand to culture the cells in an incubator at 37 °C under humidified atmosphere of 5% CO₂ and 95 % air. The medium was exchanged every 4 days. After 8 days (PAs derived from porcine-matured adipocytes) or 5 days (PAs derived from mouse-matured adipocytes) of culture, the medium containing confluent PAs was exchanged for DMEM supplemented with 50 µM hydrocortisone and 10% serum (differentiation-inducing medium), followed by culture for 10 days.

### (5) Method of identifying myoblast

The following methods identified that cultured cells were differentiated into myoblasts. That is, in order to identify myoblasts, the following were performed as indices: immunostaining with Myf5 and MyoD, which are determination factors of myoblasts; and immunostaining with a myogenin antibody, which is a determination factor of muscular cells.

### 1) Immunostaining with Myf5 and MyoD antibodies

After 4 days of induction of differentiation, cells were fixed by the following method. A 4% formalin solution in an equal amount to the differentiation-inducing medium in the culture dish was added, and the dish was allowed to stand at room temperature for 20 minutes for prefixation. After removing the prefixative solution, 2 ml of a 4% formalin solution was further added thereto, and the dish was allowed to stand at room temperature for 1 hour. After removing the fixative solution, washing was performed with phosphate buffer saline (PBS). Washing was performed with a 2% hydrogen peroxide solution in PBS three times to inhibit an intrinsic peroxidase activity. After intrinsic avidin-biotin had been inhibited, blocking was performed with PBS supplemented with normal serum for 20 minutes, and Myf5 or MyoD antibody (diluted 400-fold) was allowed to react at 4°C for 20 hours. After washing had been performed with PBS twice, a diluted biotinylated secondary antibody was allowed to react for 30 minutes, and washing was performed with PBS twice. Subsequently, ABC reagent was allowed to react for 60 minutes. After completion of the reaction, washing was performed with Tris-HCl, and DAB staining was performed for 10 minutes. Washing was performed with distilled water three times, followed by observation.

### 2) Immunostaining with myogenin antibody

PAs derived from porcine-matured adipocytes and PAs derived from mouse-matured adipocytes were fixed by the same fixation method as above after 10 to 18 days and 7 to 10 days of induction of differentiation, respectively, and washing was performed with phosphate buffer saline (PBS) three times. Washing was performed with a 2% hydrogen peroxide solution in PBS three times to inhibit an intrinsic peroxidase activity. After intrinsic avidin-biotin had been inhibited, blocking was performed with PBS supplemented with normal serum for 20 minutes, and a myogenin antibody (diluted 300-fold) was allowed to react at 4°C for 20 hours. After washing had been performed with PBS twice, a diluted biotinylated secondary antibodywas allowed to react for 30 minutes, and washing was performed with PBS twice. Subsequently, ABC reagent was allowed to react for 60 minutes. After completion of the reaction, washing was performed with Tris-HCl, and DAB staining was performed for 10 minutes. Washing was performed with distilled water three times, followed by observation.

Figs. 8 to 10 show micrographs of the myoblasts obtained by induction of differentiation of PAs derived from porcine-matured adipocytes. Meanwhile, Fig. 11 shows micrographs of myoblasts obtained by induction of differentiation of PAs derived from mouse-matured adipocytes. As is clear from those figures, it was confirmed that myoblasts can be acquired by induction of transdifferentiation of PAs derived from matured adipocytes. In most myoblasts obtained by induction of differentiation of PAs derived from porcine-matured adipocytes, markers specific to myoblasts were expressed after 18 days of induction of transdifferentiation. Meanwhile, in most myoblasts obtained by induction of differentiation of PAs derived from mouse-matured adipocytes, markers specific to myoblasts were expressed after 10 days of induction of transdifferentiation.

### (6) Induction of transdifferentiation of matured adipocyte into chondrocyte

PAs derived from porcine-matured adipocytes and PAs derived from mouse-matured adipocytes produced by the method described in (1) above were separately resuspended to 1 × 10⁵ cells/ml in a DMEM supplemented with 20% serum. Thereafter, each suspension was seeded intoaculturedish (Falcon, 3001) for tissue culture on which collagen type 1 had been coated, and the dish was allowed to stand to culture the cells in a an incubator at 37 °C under humidified atmosphere of 5% CO₂ and 95 % air. The medium was exchanged every 4 days. After 8 days (PAs derived from porcine-matured adipocytes) or 5 days (PAs derived from mouse-matured adipocytes) of culture, the medium containing confluent PAs was exchanged for a DMEM medium supplemented with 5 µg insulin, 50 µM ascorbic acid, 10 nM transforming growth factor β3, and 1% serum (differentiation-inducing medium), followed by culture for 10 to 18 days (PAs derived from porcine-matured adipocytes) and 14 days (PAs derived from mouse-matured adipocytes) . Note that, PAs that have not been subjected to induction of differentiation were used as a control, and rat-derived L6 cell line to be differentiated into chondrocytes was used as a positive control.

### (7) Method of identifying chondrocyte

The following method identified that cultured cells were differentiated into chondrocytes. That is, for identifying chondrocytes, the following were performed as indices: alcian blue staining; toluidine blue staining; and immunostaining with a collagen type 2 antibody.

### 1) Alcian blue staining

After 8 days (PAs derived from porcine-matured adipocytes) or 14 days (PAs derived from mouse-matured adipocytes) of induction of differentiation, cells were fixed by the following method, and staining was performed with alcian blue (AB). 1 ml of a 4% formalin solution was added to a differentiation-inducing medium in a culture dish, and the dish was allowed to stand at room temperature for 20 minutes for prefixation. After removing the prefixative solution, 2 ml of a 4% formalin solution was further added, and the dish was allowed to stand at room temperature for 1 hour. After removing the fixative solution, washing was performed with 2 ml of distilled water three times. 100 mg of AB was dissolved in 10 ml of 0.1N HCl, and the solution was filtrated to prepare AB staining solution. Subsequently, 2 ml of 0.1N HCl was poured in the cell-fixed culture dish, and the dish was maintained for 5 minutes at room temperature. After removing 0.1N HCl, the dish was immersed in 2 ml of AB staining solution for 30 minutes. After removing AB staining solution, washing was performed with 2 ml of distilled water three times.

### 2) Toluidine blue staining

After 8 days (PAs derived from porcine-matured adipocytes) and 14 days (PAs derived from mouse-matured adipocytes) of induction of differentiation, cells were fixed by the following method, and staining was performed with toluidine blue (TB). 1 ml of a Rossman' s fixative solution was added to a differentiation-inducing medium in a culture dish, and the dish was allowed to stand at room temperature for 20 minutes for prefixation. After removing the prefixative solution, 2 ml of a Rossman's fixative solution was further added, and the dish was allowed to stand at room temperature for 1 hour. After removing the fixative solution, washing was performed with 2 ml of distilled water three times. Subsequently, the dish was immersed in 2 ml of a 0.05% (%) TB staining solution for 60 minutes for staining. After removing the TB staining solution, washing was performed with 2 ml of distilled water three times.

### 3) Immunostaining with collagen type 2 antibody

After 16 days (PAs derived from porcine-matured adipocytes) and 14 days (PAs derived from mouse-matured adipocytes) of induction of differentiation, cells were fixed by the following method, and washing was performed with phosphate buffer saline (PBS). Washing was performed with a 2% hydrogen peroxide solution in PBS three times to inhibit an intrinsic peroxidase activity. After intrinsic avidin-biotin had been inhibited, blocking was performed with PBS supplemented with normal serum for 20 minutes, and a collagen type 2 antibody (diluted 1,000-fold) was allowed to react at 4°C for 20 hours. After washing had been performed with PBS twice, a diluted biotinylated secondary antibody was allowed to react for 30 minutes, and washing was performed with PBS twice. Subsequently, ABC reagent was allowed to react for 60 minutes. After completion of the reaction, washing was performed with Tris-HCl, and DAB staining was performed for 10 minutes. Washing was performed with distilled water three times, followed by observation.

Fig. 12 (A to D) shows micrographs of the chondrocytes obtained by induction of differentiation of control PAs and PAs derived from porcine-matured adipocytes. Meanwhile, Fig. 13 shows micrographs of chondrocytes obtained by induction of differentiation of PAs derived from mouse-matured adipocytes. AB staining, TB staining, and collagen type 2 immunostaining confirmed that, as is the case with a positive control of L6 cell line, chondrocytes can be acquired by induction of transdifferentiation of PAs derived from matured adipocytes as shown in those figures.

### (8) Induction of transdifferentiation of matured adipocyte into mammary epithelial cell

Matured adipocytes in a subcutaneous fat tissue of a transgenic mouse to which a green fluorescent protein (GFP) gene had been introduced were used to produce PA line (GFP-PA) derived from matured adipocytes by the method described (1) above. Meanwhile, mammary epithelial cells (MEs) were collected from a mammary tissue of a wild-type female mouse in the middle gestation period in accordance with a method described by Emerman et al., (Proc. Natl. Acad. Sci. USA, 74:4466-4470, 1977). That is, a mammary tissue was washed with PBS three times, and the tissue was sliced in a 0.5% (w/v) trypsin + 0.05% (w/v) EDTA solution. Subsequently, horizontal shaking was performed at 37°C for 30 minutes, and DMEM supplemented with 0.1% (w/v) type I collagenase and 5% FCS (v/v) was added, followed by stirring at 37°C for 45 minutes (100 to 120 times/minute). Thereafter, the cell suspension was centrifuged (× 200 g, for 1 minute) to remove the supernatant, and 10 ml of DMEM supplemented with 10% FCS was added to resuspend the cells. The same centrifugation-washing treatment was repeated three times to remove hemocytes and fibroblasts. The cell suspension was filtrated with a 150 µm mesh to remove non-digestive tissues, and the finally obtained mammary epithelial cells were used for culture. The resultant mammary epithelial cells, GFP-PA, and ME were resuspended in a DMEM supplemented with 20% serum, and three-dimensional culture was performed in type 1 collagen (1.5%). After the cells were cultured in a DMEM supplemented with 20% serum until the second day, the medium was exchanged for a medium supplemented with 5.0 mg/ml bovine serum albumin, 5 µg/ml prolactin, 1 µg/ml dexamethasone, 0.01% (v/v) ITS, and 10 mM Hepes (differentiation-inducing medium), and the dish was allowed to stand to culture the cells for 2 weeks in an incubator at 37 °C under humidified atmosphere of 5% CO₂ and 95 % air. After 2 weeks of culture, collagen was peeled from the bottom surface of the culture dish to suspend the cells, and culture was performed for additional 2 weeks.

### (9) Method of identifying mammary epithelial cell

The following method identified that co-cultured GFP-PA was transdifferentiated into ME. That is, although a cell in which GFP was expressed is a major premise to identify ME derived from GFP-PA, immunostainings with antibodies of E-cadherin, vinculin, keratin, and ZO-1 specifically expressed in an epithelial cell were performed as indices. Note that, wild-type ME was used as a control.

### 1) Immunostaining with E-cadherin, vinculin, keratin, and ZO-1 antibodies

After 28 days of induction of differentiation, collagen gel in the culture dish was taken out and washed with PBS, and the gel was embedded with O.T.C. compound for preparing frozen sections (Tissue Tek.) in accordance with the conventional method. Thereafter, 0.5 µm of frozen serial sections were prepared by a cold tome. The sections were immersed in a 4% formalin solution and allowed to stand at room temperature for 1 hour for fixation, and washing was performed with phosphate buffered saline (PBS) three times. The sections were immersed in PBS supplemented with 0.1% (v/v) Tween 20 (T-PBS) for 5 minutes, and blocking was performed with PBS supplemented with 1.5% (v/v) rabbit serum for 60 minutes. Thereafter, E-cadherin, vinculin, keratin, and ZO-1 antibodies diluted to various concentrations (diluted 200 to 1, 000-fold) each were allowed to react at 4°C for 18 hours. A TRITC labeled-mouse antibody diluted 200-fold was allowed to react at room temperature for 30 minutes. Subsequently, washing was performed with PBS twice under shade, and the specimens were air-dried, followed by observation using an optical microscope or a fluorescent microscope.

Figs. 14 to 17 show the micrographs of MEs derived from matured adipocytes. GFP-PAs assembled in three-dimensional culture to form a tubular structure (Fig. 17), and they had torus-shape alveolar structures each having an alveolar lumen in the center similar to a positive control (Fig. 16) and had epithelial cell-like shapes.

As a result of the immunostaining, GFP-PAs having epithelial cell-like shapes were stained with E-cadherin, vinculin, keratin, and ZO-1 antibodies (Figs. 16 and 17) . As is clear from those figures, it was confirmed that GFP-PAs derived from matured adipocytes were transdifferentiated into MEs to form a mammary alveoli.

### (10) Induction of transdifferentiation of matured adipocyte into neurocyte

PAs derived from porcine-matured adipocytes and PAs derived from mouse-matured adipocytes produced by the method described in (1) above, were separately resuspended in DMEM supplemented with 20% serum (for porcine cells) and in a DMEM supplemented with 10% serum (for mouse cells) to 1 × 10⁵ cells/ml. Thereafter, each suspension was inoculated in a culture dish (Falcon, 3001) or a flask (Falcon, 3107) for tissue culture on which collagen type 1 or 3 had been applied, and the dish or flask was allowed to stand to culture the cells in an incubator at 37 °C under humidified atmosphere of 5% CO₂ and 95 % air. Transdifferentiation into neurocytes was performed according to the method described by Woodbury et al., (J. Neuro. Res., 61: 364-370 2000). That is, after 6 to 7 days of culture, the medium containing 80% confluent porcine PAs was exchanged for a DMEM supplemented with 1-10 mM β-mercaptoethanol (BME) and 20% FCS, while the medium containing mouse PAs was exchanged for a DMEM supplemented with 1-10 mM BMEand 10% FCS, followed by culture for 12 hours respectively. Washing was performed with PBS, and culture was performed for additional 5 hours in a DMEM supplemented with 1 mM BME to perform induction of transdifferentiation into neurocytes. Note that, PAs that have not been subjected to induction of differentiation were used as a control, while Ng108-15 cell line to be differentiated into neurocytes was used as a positive control.

### (11) Method of identifying neurocyte

The following method identified that cultured PAs were differentiated into neurocytes. That is, for identifying neurocytes, immunostaining was performed with nestin, neuron-specific enolase, βIII-tubulin, MAP2 (Microtubule-associated protein 2), and neurofilament antibodies as indices.

After culture, 1 ml of a 4% formalin solution was added to a differentiation-inducing medium in a culture dish after 12 hours of induction of differentiation, and the dish was allowed to stand at room temperature for 20 hours for prefixation. After removing the prefixative solution, 2 ml of a 4% formalin solution was further added, and the dish was allowed to stand at room temperature for 1 hour, followed by washing with phosphate buffer saline (PBS). Washing was performed with 2% hydrogen peroxide-PBS three times to inhibit an intrinsic peroxidase activity. After intrinsic avidin-biotin had been inhibited, blocking was performed with PBS supplemented with normal serum for 20 minutes, and nestin, neuron-specific enolase, βIII-tubulin, and neurofilament antibodies (diluted 200 to 1, 000-fold) each were allowed to react at 4°C for 20 hours. After washing had been performed with PBS twice, a diluted biotinylated secondary antibody was allowed to react for 30 minutes, and washing was performed with PBS twice. Subsequently, ABC reagent was allowed to react for 60 minutes. After completion of the reaction, washingwasperformedwithTris-HCl, andDABstaining was performed for 10 minutes. Washing was performed with distilled water three times, and counter staining was performed with hematoxylin in accordance with the conventional method, followed by observation.

Figs. 18 and 19 show micrographs of the nerves derived from porcine-matured adipocytes. Meanwhile, Fig.20 shows micrographs of nerves derived from mouse-matured adipocytes. As a result of induction of differentiation of porcine PAs and mouse PAs into neurocytes, those cells were found to have neurocyte-like shapes (Figs. 18 and 20). As a result of the immunostaining, PAs having neurocyte-like shapes were stained with nestin, neuron-specific enolase, βIII-tubulin, MAP2, and neurofilament antibodies (Fig. 19) as is the case with a control. As is clear from those figures, it was confirmed that neurocytes can be obtained by induction of transdifferentiation of porcine PAs and mouse PAs derived from matured adipocytes.

### [Industrial Applicability]

The effects of the present invention will be listed below.
(1) The present invention is the only method of clarifying transdifferentiation mechanism to acquire osteoblasts, myoblasts, chondrocytes, epithelial cells, and neurocytes by induction of transdifferentiation of PAs. It has been known that adipocytes, osteocytes, muscular cells, and osteoblasts are originated from the same mesodermal stem cells, while neurocytes and epithelial cells are derived from ectodermal stem cells. And it has been considered that, on the differentiation directionalities, terminal differentiation is caused from the stem cells via respective precursor cells into adipocytes or osteocytes. The present invention defies those common senses and serves the only method of acquiring osteoblasts, myoblasts, chondrocytes, epithelial cells, and neurocytes by induction of transdifferentiation of PAs obtained by dedifferentiation of matured adipocytes,tothereby significantly contribute to clarification of transdifferentiation mechanism.
(2) The resultant cells have the following effects as donor cells for novel regenerative medicine.
   1) Collection is easily performed because subcutaneous fats are used, and donors carry less risk of anesthesia and have fewer burdens.
   2) Many matured adipocyte numbers are obtained because many cells are collected, and many osteoblasts, myoblasts, chondrocytes, epithelial cells, and neurocytes can be obtained in proportion to the numbers. Therefore, osteoblasts, myoblasts, chondrocytes, epithelial cells, and neurocytes can be collected at significantly low cost.
   3) Maturedadipocytes canbe collectedas a single cell fraction owing to their structures, so that a uniform cell group can be separately collected easily without a special apparatus.
   4) PAs derived from matured adipocytes are fibroblasts, so that they are easily treated and require no special culture technique.
   5) subcutaneous fats exist in from a neonate to a senior, so that the present invention can be performed despite the age of a subject to be treated.
   6) The present invention can be preformed without using a fertilized ovum, so that it is not subjected to ethical constraints. In addition, wastes discharged in esthetic surgery and the like can be reused.

### [Reference to deposited biological material]

### (I) Name and address of a depository institution where the biological material is deposited

Name: International Patent Organism Depositary,

### National Institute of Advanced Industrial Science and Technology

Address: Central 6, Higashi 1-1-1, Tsukuba, Ibaraki, Japan (II) Date deposited to the institute (I)

February 20, 2004 (original deposit date under Budapest Treaty) (III) Deposit No. assigned by the institute (I) for the deposit FERM BP-08645

## Claims

1. A cell culture method comprising the steps:
a) obtaining a fraction of unilocular mature adipocytes derived from subcutaneous fat tissue sample obtained from a subject by filtration of the tissue sample, subjecting the fraction to centrifugation and recovering the unilocular adipocytes separated in the upper layer;
b) subjecting said mature adipocytes to ceiling culture and continuing the culture for at least a further 14 days with the cell adhered-surface at the bottom to obtain ffbroblast-like preadipocytes having no lipid droplets, which act as preadipocytes expressing an early marker of osteogenesis, myogenesis or adipogenesis:
c) inducing transdifferentiation of said preadipocytes by culturing using differentiation-inducing medium to give a transdifferentiated cell selected from an osteoblast, a myoblast, a chondrocyte, a mammary epithelial cell and a neurocyte.

2. A cell culture method comprising the step:
inducing transdifferentiation, by culturing using differentiation-inducing medium, of the preadipocyte cell line deposited as FERM BP-08845 to give a transdifferentiated cell selected from an osteoblast, a myoblast, a chondrocyte, a mammary epithelial cell and a neurocyte.

3. A method according to any one of claims 1 to 2, wherein the transdifferentiated cell is an osteoblast

4. A method according to any one of claims 1 to 2, wherein the transdifferentiated cell is a myoblast.

5. A method according to any one of claims 1 to 2. wherein the transdifferentiated cell is a chondrocyte.

6. A method according to any one of claims 1 to 2, wherein the transdifferentiated cell is a mammary epithelial cell.

7. A method according to any one of claims 1 to 2, wherein the transdifferentiated cell is a neurocyte.

## Patentansprüche

1. Zellkulturverfahren, das die folgenden Schritte beinhaltet:
a) Erlangen einer Fraktion unilokulärer reifer Adipozyten, die von einer subkutanen Fettgewebeprobe abstammen, die von einem Subjekt erhalten wurde, durch Filtrieren der Gewebeprobe, Aussetzen der Fraktion einer Zentrifugation und Gewinnen der in der oberen Lage abgeschiedenen unilokulären Adipozyten;
b) Aussetzen der genannten reifen Adipozyten einer Deckenkultur und Fortsetzen der Kultur über einen Zeitraum von wenigstens weiteren 14 Tagen mit der Zelladhäsionsfläche am Boden, um fibroblastenartige Präadipozyten zu erhalten, die keine Lipidtropfen haben, die als Präadipozyten wirken, die einen frühen Marker von Osteogenese, Myogenese oder Adipogenese exprimieren;
c) Induzieren einer Transdifferenzierung der genannten Präadipozyten durch Kultivieren mittels eines differenzierungsinduzierenden Mediums, um eine transdifferenzierte Zelle zu erhalten, die ausgewählt ist aus einem Osteoblasten, einem Myoblasten, einem Chondrozyten, einer Säugetierepithelzelle und einem Neurozyten.

2. Zellkulturverfahren, das die folgenden Schritte beinhaltet:
Induzieren einer Transdifferenzierung der Präadipozytenzelllinie, die als FERM BP-08645 hinterlegt ist, durch Kultivieren mittels eines differenzierungsinduzierenden Mediums, um eine transdifferenzierte Zelle zu erhalten, die ausgewählt ist aus einem Osteoblasten, einem Myoblasten, einem Chondrozyten, einer Säugetierepithelzelle und einem Neurozyten.

3. Verfahren nach einem der Ansprüche 1 bis 2, wobei die transdifferenzierte Zelle ein Osteoblast ist.

4. Verfahren nach einem der Ansprüche 1 bis 2, wobei die transdifferenzierte Zelle ein Myoblast ist.

5. Verfahren nach einem der Ansprüche 1 bis 2, wobei die transdifferenzierte Zelle ein Chondrozyt ist.

6. Verfahren nach einem der Ansprüche 1 bis 2, wobei die transdifferenzierte Zelle eine Säugetierepithelzelle ist.

7. Verfahren nach einem der Ansprüche 1 bis 2, wobei die transdifferenzierte Zelle ein Neurozyt ist.

## Revendications

1. Procédé de culture cellulaire comprenant les étapes consistant à :
a) obtenir une fraction d'adipocytes matures uniloculaires dérivée d'un échantillon de tissu adipeux sous-cutané d'un sujet par filtration de l'échantillon tissulaire, soumettre la fraction à la centrifugation et recueillir les adipocytes uniloculaires séparés dans la couche supérieure;
b) soumettre lesdits adipocytes matures à une culture dite "ceiling" et continuer la culture pendant au moins 14 jours de plus avec la surface collée aux cellules au fond pour obtenir des préadipocytes genre fibroblastes sans gouttelette lipidique, lesquels agissent comme des préadipocytes exprimant un marqueur précoce de l'ostéogenèse, de la myogenèse ou de l'adipogenèse;
c) induire la transdifférenciation desdits préadipocytes par culture en utilisant un milieu inducteur de différenciation pour donner une cellule transdifférenciée sélectionnée parmi un ostéoblaste, un myoblaste, un chondrocyte, une cellule épithéliale mammaire et un neurocyte.

2. Procédé de culture cellulaire comprenant l'étape consistant à :
induire la transdifférenciation, par culture en utilisant un milieu inducteur de différenciation, de la lignées cellulaire de préadipocytes déposée sous le n° FERM BP-08645, pour donner une cellule transdifférenciée sélectionnée parmi un ostéoblaste, un myoblaste, un chondrocyte, une cellule épithéliale mammaire et un neurocyte.

3. Procédé selon l'une quelconque des revendications 1 à 2, dans lequel la cellule transdifférenciée est un ostéoblaste.

4. Procédé selon l'une quelconque des revendications 1 à 2, dans lequel la cellule transdifférenciée est un myoblaste.

5. Procédé selon l'une quelconque des revendications 1 à 2, dans lequel la cellule transdifférenciée est un chondrocyte.

6. Procédé selon l'une quelconque des revendications 1 à 2, dans lequel la cellule transdifférenciée est une cellule épithéliale mammaire.

7. Procédé selon l'une quelconque des revendications 1 à 2, dans lequel la cellule transdifférenciée est un neurocyte.
